# EUROPEAN PATENT APPLICATION

(11) **EP 3 315 151 A1**
(43) Date of publication of application: **02.05.2018**
(21) Application number: 16196759.1
(22) Date of filing: 01.11.2016
(51) Int. Cl.: A61M 5/00

(54) **KIT FOR ADMINISTRATION OF MEDICAMENTS**

(71) Applicant: MEDA Pharma GmbH & Co. KG, 61352 Bad Homburg (DE)
(72) Inventor: Tritschler, Hans-Jürgen, 80639 München (DE); Gehenio, Dagmar, 45481 Mühlheim (DE); Fernandez Saez, Carlos, 28022 Madrid (ES)
(74) Representative: Mullen, Lee Bryan

(57) **Abstract**

The present invention relates to a kit for administration of medicaments. The kit comprises a box (1) that contains at least one medicament delivery device (4, 4a) and a video device (3). The video device (3) may be configured to provide visual or audio-visual instructions for using the medicament delivery device (4, 4a). Both the at least one medicament delivery device (4, 4a) and the video device (3) are removably attached to the box (1). Removal of the at least one medicament delivery device (4, 4a) and the video device (3) from the box (1) allows carrying the at least one medicament delivery device (4, 4a) and the video device (3) without the need to carry the entire box (1). As carrying the at least one medicament delivery device (4, 4a) and the video device (3) without the box (1) is much more comfortable as compared to carrying the entire bulky box (1) it is more likely as compared to the prior art that the patient will indeed carry the medicament delivery device (4, 4a) at all times so that the medicament delivery device (4, 4a) is available when needed. Moreover, as the video device can be removed from the bulky box (1) it is more likely as compared to the prior art that the patient will also carry the video device (3). Thus, the likelihood of improper use of the medicament delivery device (4, 4a) is decreased as compared to the prior art.

## Description

Self-administered medicament delivery devices, such as, for example pre-filled medical injectors, inhalers, transdermal delivery devices and the like are often used as a part of a patient's medication regimen. For example, known self-administered medicament delivery devices can be used as a part of a patient's emergency care regimen. Emergency care regimens can include, for example, using an auto-injector to rapidly self-administer a medicament in response to an allergic reaction, for example epinephrine, or for the treatment of other emergency conditions as for example poisoning. Known self-administered medicament delivery devices can also be used as a part of a patient's chronic care regimen. Chronic care regimens can include, for example, using a pen injector to self-administer a medicament according to a prescribed plan. Examples of chronic care regimens can include, for example, the injection of insulin, the injection of human growth hormone (HGH), erythropoiesis-stimulating agents (ESA), Interferons and other chronic therapies, or the like. Furthermore, self-administered medicament delivery devices can also be used for preventive/prophylactic therapies. Examples of preventive/prophylactic therapies include certain vaccines, such as an influenza vaccine.

To actuate some known medicament delivery devices, the user may be required to execute a series of operations. For example, to actuate some known auto-injectors, the user must remove a protective cap, remove a locking device, place the auto-injector in a proper position against the body and then press a button to actuate the auto-injector. Failure to complete these operations properly can result in an incomplete injection and/or injection into an undesired location of the body.

The likelihood of improper use of known medicament delivery devices can be compounded by the nature of the user and/or the circumstances under which such devices are used. For example, many users are not trained medical professionals and may have never been trained in the operation of such devices. Moreover, in certain situations, the user may not be the patient, and may therefore have no experience with the medicament delivery device. Similarly, because some known medicament delivery devices are configured to be used relatively infrequently in response to an allergic reaction or the like, even those users familiar with the device and/or who have been trained may not be well practiced at operating the device. Finally, such devices often have to be used during an emergency situation, during which even experienced and/or trained users may be subject to confusion, panic and/or the physiological effects of the condition requiring treatment. It is essential that the person who needs treatment can calm down and stop any panic by quick and safe administration of the required medication. It should also be possible that people who become aware of the emergency situation can help the patient. In a best case scenario even elderly people and children should be in the position to help the patient.

It has been suggested in US 9,278,177 B2 that the likelihood of improper use of known medicament delivery devices can be substantially reduced if a medicament delivery device is provided together with an output device that provides an audio-visual output via a speaker and an LCD screen showing step-by-step instructions for using the medicament delivery device.

US 9,278,177 B2 suggests that a medicament delivery device and an output device are provided together as a medical system in form of a box that contains both the medicament delivery device and the output device. The medicament delivery device is contained in an interior cavity of the box that is hidden under a hinged lid. The lid can be opened in order to remove the medicament delivery device from the box. The lid comprises an LCD screen as part of its internal surface. Thus, the screen becomes visible to the user upon opening of the lid. Once the medicament delivery device is removed from the box, a switch in the box is moved in a position such that the audio-visual instruction for using the medicament delivery device are provided. The speakers for providing the audio output are not part of the hinged lid but are integrated into the rear surface of the box. Importantly, both the speakers and the LCD screen are permanently connected to the box and cannot be removed from the box. Furthermore, the box comprises all necessary electronic equipment for providing the audio-visual instructions upon removal of the medicament delivery device. Thus, according to the system suggested by US 9,278,177 B2 the patient has to carry the entire box in order to be able to receive the audio-visual instructions for using the medicament delivery device.

However, this is disadvantageous because such a box is bulky and carrying it is inconvenient and undesirable. Accordingly, the patient may not carry the medicament delivery device at all times so that the medicament delivery device may not be available when needed. Furthermore, the patient may remove the medicament delivery device from the box in order to carry only the medicament delivery device and to avoid carrying the bulky box. However, in such a case the audio-visual instructions for using the medicament delivery device may not be available when needed so that the likelihood of improper use of the medicament delivery device is increased.

The problems of the prior art are solved by the subject-matter of the patent claims. The problems are in particular solved by a kit comprising a box (1) containing at least one medicament delivery device (4, 4a) and a video device (3), wherein both the at least one medicament delivery device (4, 4a) and the video device (3) are removably attached to the box (1).

The kit of the present invention comprises a box (1). The box (1) is useful for compactly combining the at least one medicament delivery device (4, 4a) and the video device (3). Suitable is in principle any kind of box (1) that is large enough for containing the at least one medicament delivery device (4, 4a) and the video device (3). The box (1) is preferably made from a robust material in order to prevent damage to the at least one medicament delivery device (4, 4a) and/or the video device (3) by external forces. Preferred materials are selected from the group consisting of paperboard, plastics and metal.

Preferably, the box (1) of the present invention is closable. Thus, the box (1) is preferably reversibly switchable from an open state to a closed state and from a closed state to an open state. Preferably, the box (1) comprises a lid (2) configured for closing the box (1). Preferably, the lid (2) is movable between an open position, in which access to the at least one medicament delivery device (4, 4a) and to the video device (3) in the box (1) is possible, and a closed position, in which access to the at least one medicament delivery device (4, 4a) and to the video device (3) in the box (1) is prevented by the lid (2). Preferably, the lid (2) is movable between the open position and the closed position via a hinge that connects the lid (2) to the corpus of the box. Preferably, the lid (2) comprises closing means (2a) for fixing the lid (2) in the closed position by detachably attaching the lid (2) to the corpus of the box (1). Preferably, the closing means (2a) are located on the opposite side of the hinge. Preferably, the closing means (2a) are configured for interaction with corresponding closing means on the corpus of the box (1) so that the closing means (2a) on the lid (2) and the corresponding closing means on the corpus of the box (1) form a closing mechanism. Preferred closing mechanisms are selected from the group consisting of magnetic locks, zip fasteners and hook-and-loop fasteners.

The box (1) contains at least one medicament delivery device (4, 4a). Preferably, the box (1) contains exactly two medicament delivery devices (4, 4a), preferably a first medicament delivery device (4) and a second medicament delivery device (4a). Preferably, the first medicament delivery device (4) and the second medicament delivery device (4a) are interchangeable. Preferably, the medicament delivery devices (4, 4a) of the invention are configured for self-administration of medicaments. Preferred medicament delivery devices (4, 4a) are selected from the group consisting of pre-filled medical injectors, auto-injectors, inhalers, and transdermal delivery devices.

Preferably, the medicament delivery device (4, 4a) comprises at least one active ingredient. Preferred active ingredients are selected from the group consisting of epinephrine, insulin, human growth hormone (HGH), erythropoiesis-stimulating agents (ESA), interferons and vaccines, such as an influenza vaccine. More preferably, the active ingredient is selected from the group consisting of epinephrine, insulin, human growth hormone (HGH) and erythropoiesis-stimulating agents (ESA).

The box (1) also contains a video device (3). The video device (3) is preferably configured to provide visual or audio-visual instructions for using the medicament delivery device (4, 4a). Audio-visual instructions are preferred. The instructions are preferably step-by-step instructions for using the medicament delivery device (4, 4a). Instructions for using the medicament delivery device (4, 4a) are useful for reducing the likelihood of improper use of the medicament delivery device (4, 4a).

Preferably, the video device (3) is comparably small. A comparably small video device (3) can be conveniently carried. Preferably, the size of the video device (3) is comparable to the size of the medicament delivery device (4, 4a). This allows that the video device (3) and the medicament delivery device (4, 4a) can be conveniently carried together. Preferably, the length of the video device (3) is at most 200%, more preferably at most 150%, more preferably at most 125% more preferably at most 110% of the length of the medicament delivery device (4, 4a). Preferably, the width of the video device (3) is at most 200%, more preferably at most 150%, more preferably at most 125% more preferably at most 110% of the width of the medicament delivery device (4, 4a). Preferably, the thickness of the video device (3) is at most 200%, more preferably at most 150%, more preferably at most 125% more preferably at most 110% of the thickness of the medicament delivery device (4, 4a). Preferably, the length of the video device (3) is at most 20 cm, more preferably at most 15 cm, more preferably at most 12 cm, more preferably at most 10 cm. Preferably, the width of the video device (3) is at most 10 cm, more preferably at most 8 cm, more preferably at most 6 cm, more preferably at most 5 cm. Preferably, the thickness of the video device (3) is at most 2 cm, more preferably at most 1.5 cm, more preferably at most 1.2 cm, more preferably at most 1 cm, more preferably at most 0.8 cm, more preferably at most 0.5 cm, more preferably at most 0.2 cm. Preferably, the dimensions of the video device (3) are at most 20x10x2 cm, more preferably, at most 15x8x1.5 cm, more preferably at most 12x6x1 cm, more preferably at most 10x5x0.5 cm. For example, dimensions of at most 20x10x2 cm means according to the present description that the video device (3) has a length of at most 20 cm, a width of at most 10 cm and a thickness of at most 2 cm.

Both the at least one medicament delivery device (4, 4a) and the video device (3) are removably attached to the box (1). Removal of the at least one medicament delivery device (4, 4a) and the video device (3) from the box (1) allows carrying the at least one medicament delivery device (4, 4a) and the video device (3) without the need to carry the entire box (1). As carrying the at least one medicament delivery device (4, 4a) and the video device (3) without the box (1) is much more comfortable as compared to carrying the entire bulky box (1) it is more likely as compared to the prior art that the patient will indeed carry the medicament delivery device (4, 4a) at all times so that the medicament delivery device (4, 4a) is available when needed. Moreover, as the video device can be removed from the bulky box (1) it is more likely as compared to the prior art that the patient will also carry the video device (3) so that the instructions for using the medicament delivery device (4, 4a) are available when needed. Thus, the likelihood of improper use of the medicament delivery device (4, 4a) is decreased as compared to the prior art.

Preferably, the at least one medicament delivery device (4, 4a) and the video device (3) are attached to the box (1) via holding means. Preferably, the holding means are part of the box (1). The holding means (2b) for the video device (3) are preferably located at the internal surface of the lid (2).

Preferably, the at least one medicament delivery device (4, 4a) and the video device (3) are attached to the box (1) in such a way that access to the at least one medicament delivery device (4, 4a) and to the video device (3) is prevented when the lid (2) is in the closed position. In such a way, the at least one medicament delivery device (4, 4a) and the video device (3) are protected from external forces if the lid (2) is in the closed position.

Preferably, the at least one medicament delivery device (4, 4a) and the video device (3) are removably attached to the box (1) in such a way that the at least one medicament delivery device (4, 4a) and the video device (3) can be removed from the box (1) independently from each other. In such a way, the patient can decide whether the at least one medicament delivery device (4, 4a) or the video device (3) or both the at least one medicament delivery device (4, 4a) and the video device (3) should be carried. For example, for patients that are highly experienced with self-administration of the medicament there is no need to carry the video device (3). On the other hand, it may be advantageous if the video device (3) can be carried separately because it may then be used for training purposes without the need to carry the expensive and potentially sensitive medicament delivery device (4, 4a). Furthermore, it may be disadvantageous for both receiving the instructions provided by the video device (3) and for administering the medicament by use of the medicament delivery device (4, 4a) if the video device (3) and the medicament delivery device (4, 4a) were inseparably connected to each other.

Preferably, the box (1) further comprises a medicament delivery device dummy (6). Such dummy (6) may be useful for testing the way of applying the medicament delivery device (4, 4a). Preferably, the medicament delivery device dummy (6) is constructed analogously to the medicament delivery device (4, 4a). However, the medicament delivery device dummy (6) does not contain any medicament in contrast to the medicament delivery device (4, 4a). Thus, the patient may use the medicament delivery device dummy (6) according to the instructions provided by the video device (3) in order to practice the self-administration of the medicament.

Preferably, the box (1) further comprises a removable casing (5) for the at least one medicament delivery device (4, 4a) and/or the video device (3). Preferably, the casing (5) is a bag (5). Preferably, the casing (5) comprises a first compartment (9) for receiving the at least one medicament delivery device (4, 4a) and a second compartment (10) for receiving the video device (3). This allows that the video device (3) and the medicament delivery device (4, 4a) can be conveniently carried together.

Preferably, the first compartment (9) is closable. Thus, the first compartment (9) is preferably reversibly switchable from an open state to a closed state and from a closed state to an open state. Preferably, the first compartment (9) comprises a lid configured for closing the first compartment (9). Preferably, the lid is movable between an open position, in which access to the at least one medicament delivery device (4, 4a) in the first compartment (9) is possible, and a closed position, in which access to the at least one medicament delivery device (4, 4a) in the first compartment (9) is prevented by the lid. Preferably, the lid is movable between the open position and the closed position via a hinge that connects the lid to the corpus of the casing (5). Preferably, the casing (5) comprises closing means (7) for reversibly fixing the lid in the closed so that the first compartment (9) is in the closed state. Preferred closing means (7) are selected from the group consisting of magnetic locks, zip fasteners and hook-and-loop fasteners. Particularly preferred closing means (7) are zip fasteners.

Preferably, the second compartment (10) is closable. Thus, the second compartment (10) is preferably reversibly switchable from an open state to a closed state and from a closed state to an open state. Preferably, the casing (5) comprises closing means (8) configured for reversibly closing the second compartment (10), thus for reversibly fixing the second compartment (10) in the closed state. Preferably, the closing means (8) are movable between an open position, in which access to the video device (3) in the second compartment (10) is possible, and a closed position, in which access to the video device (3) in the second compartment (10) is prevented by the closing means (8). Preferred closing means (8) are selected from the group consisting of elastic bands, magnetic locks, zip fasteners and hook-and-loop fasteners. Particularly preferred closing means (8) are elastic bands and magnetic locks. Even more preferred closing means (8) are elastic bands.

In preferred embodiments, both the first compartment (9) and the second compartment (10) are closable.

The box (1) of the present invention may also comprise both a medicament delivery device dummy (6) and a removable casing (5) for the at least one medicament delivery device (4, 4a) and/or the video device (3).

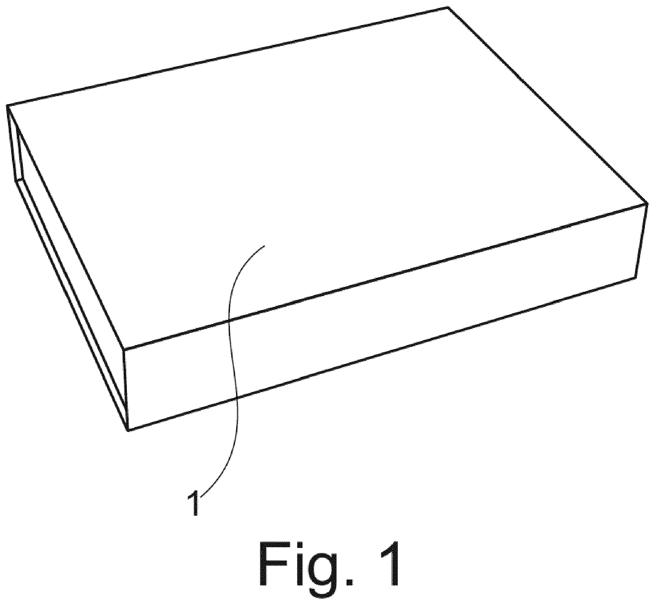

The present invention shall be further explained with regard to preferred embodiments shown in figures 1 to 5. It is understood that the present invention is not restricted to the embodiments shown in the figures. Rather, these embodiments are preferred and representative embodiments that exemplarily illustrate the inventive concept of the present invention.
Figure 1 shows a perspective scheme of a box (1) of the present invention. In figure 1, the box (1) is in a closed state that does not allow direct access to the at least one medicament delivery device (4, 4a) and the video device (3) contained in the box (1). The box (1) has to be opened in order to get access to the at least one medicament delivery device (4, 4a) and the video device (3).

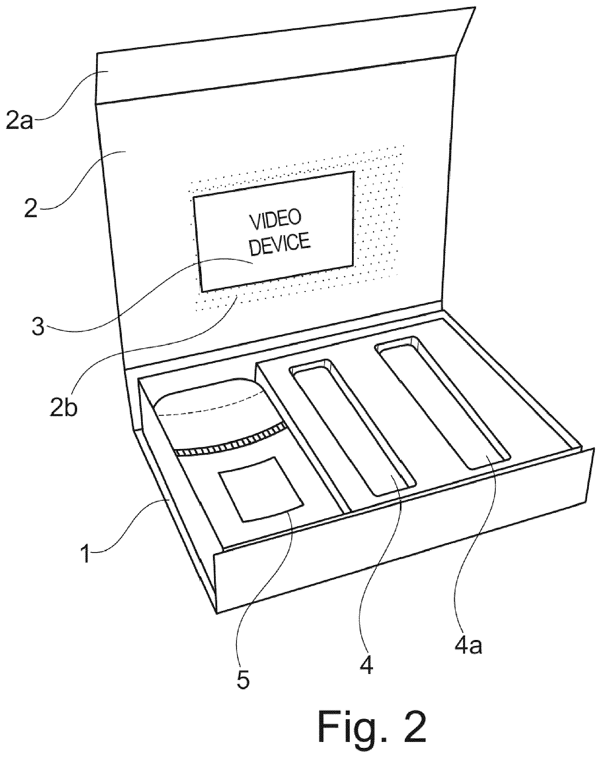

Figure 2 shows a perspective scheme of a box (1) of the present invention. Figure 2 shows a preferred embodiment according to the present invention. Figure 2 shows the box (1) in an open state. Thus, the lid (2) is in an open position so that access to the medicament delivery devices (4, 4a) and to the video device (3) in the box (1) is possible. According to the embodiment shown in figure 2, the box (1) comprises exactly two medicament delivery devices (4, 4a). The video device (3) is attached to the box (1) via holding means (2b). The holding means (2b) for the video device (3) are located at the internal surface of the lid (2). The lid (2) is movable between the open position and the closed position via a hinge that connects the lid (2) to the corpus of the box. The lid (2) comprises closing means (2a) for fixing the lid (2) in the closed position by detachably attaching the lid (2) to the corpus of the box (1). The closing means (2a) are located on the opposite side of the hinge. The box (1) further comprises a removable casing (5) for the medicament delivery devices (4, 4a) and for the video device (3).

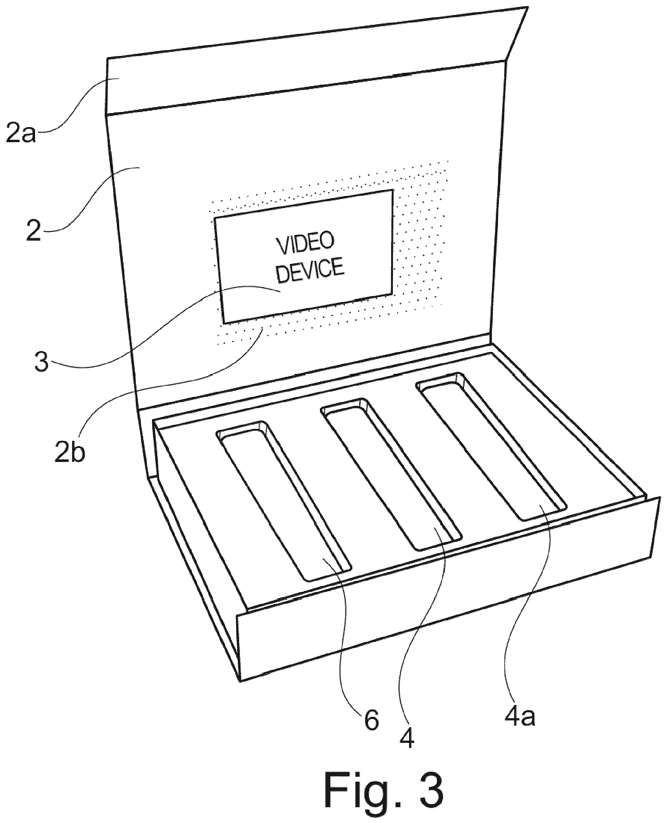

Figure 3 shows a perspective scheme of a box (1) of the present invention. Figure 3 shows the box (1) in an open state. Figure 3 shows a preferred embodiment according to the present invention. Figure 3 shows an alternative embodiment as compared to the embodiment shown in figure 2. The box (1) shown in figure 3 comprises a medicament delivery device dummy (6).

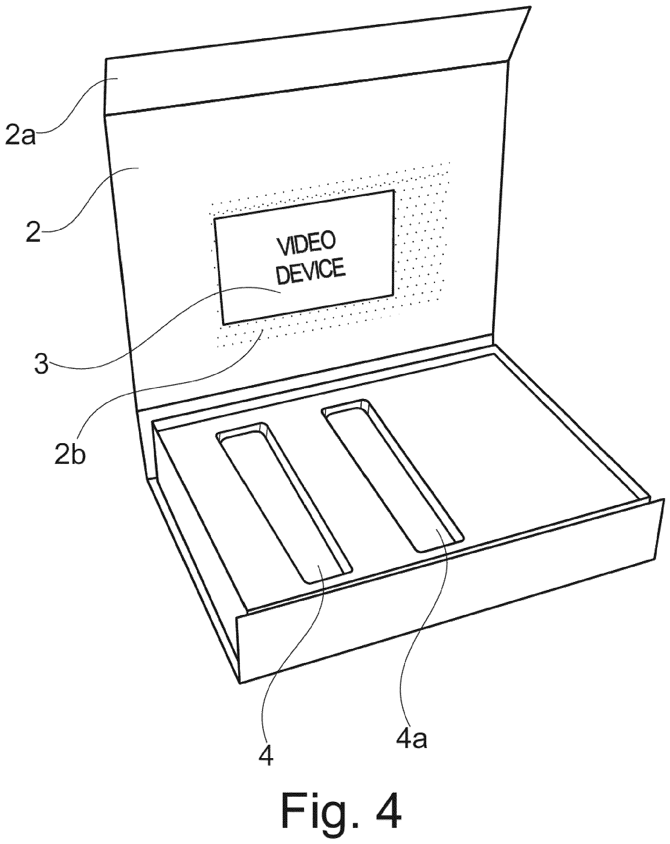

Figure 4 shows a perspective scheme of a box (1) of the present invention. Figure 4 shows the box (1) in an open state. Figure 4 shows a preferred embodiment according to the present invention. Figure 4 shows an alternative embodiment as compared to the embodiments shown in figures 2 and 3. The box (1) shown in figure 4 does neither comprise a medicament delivery device dummy (6) nor a removable casing (5) for the medicament delivery devices (4, 4a) and for the video device (3).

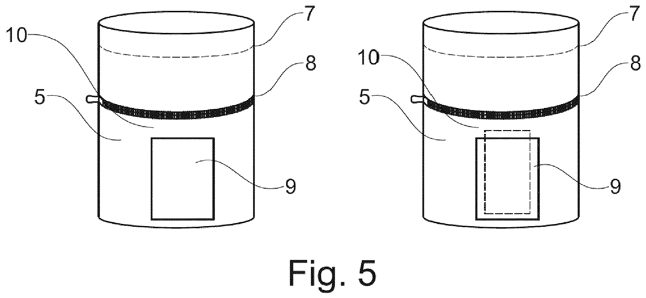

Figure 5 shows a perspective scheme of a removable casing (5) for the at least one medicament delivery device (4, 4a) and for the video device (3). The casing (5) comprises a first compartment (9) for receiving the at least one medicament delivery device (4, 4a) and a second compartment (10) for receiving the video device (3). The casing (5) comprises closing means (7) for reversibly closing the first compartment (9). The casing (5) also comprises closing means (8) for reversibly closing the second compartment (10).

### List of reference signs

- 1: box
- 2: lid
- 2a: closing means
- 3: video device
- 4, 4a: medicament delivery device
- 5: casing
- 6: medicament delivery device dummy
- 7: closing means
- 8: closing means
- 9: first compartment
- 10: second compartment

## Claims

1. Kit comprising a box (1) containing at least one medicament delivery device (4, 4a) and a video device (3), wherein both the at least one medicament delivery device (4, 4a) and the video device (3) are removably attached to the box (1).

2. Kit according to claim 1, wherein the box (1) is closeable by a lid (2) that is movable between an open position and a closed position.

3. Kit according to claim 2, wherein the lid (2) comprises closing means (2a) for fixing the lid (2) in the closed position by detachably attaching the lid (2) to the corpus of the box (1).

4. Kit according to claim 3, wherein the closing means (2a) are configured for interaction with corresponding closing means on the corpus of the box (1) so that the closing means (2a) on the lid (2) and the corresponding closing means on the corpus of the box (1) form a closing mechanism selected from the group consisting of magnetic locks, zip fasteners and hook-and-loop fasteners.

5. Kit according to at least one of the preceding claims, wherein the at least one medicament delivery device (4, 4a) and the video device (3) are removably attached to the box (1) in such a way that access to the at least one medicament delivery device (4, 4a) and to the video device (3) is prevented when the lid (2) is in the closed position.

6. Kit according to at least one of the preceding claims, wherein the at least one medicament delivery device (4, 4a) and the video device (3) are removably attached to the box (1) in such a way that the at least one medicament delivery device (4, 4a) and the video device (3) can be removed from the box (1) independently from each other.

7. Kit according to at least one of the preceding claims, wherein the box (1) contains a first medicament delivery device (4) and a second medicament delivery device (4a).

8. Kit according to at least one of the preceding claims, wherein the at least one medicament delivery device (4, 4a) is selected from the group consisting of pre-filled medical injectors, auto-injectors, inhalers, and transdermal delivery devices.

9. Kit according to at least one of the preceding claims, wherein the at least one medicament delivery device (4, 4a) comprises at least one active ingredient.

10. Kit according to at least one of the preceding claims, wherein the active ingredient is selected from the group consisting of epinephrine, insulin, human growth hormone (HGH) and erythropoiesis-stimulating agents (ESA).

11. Kit according to at least one of the preceding claims, wherein the dimensions of the video device (3) are at most 20x10x2 cm, preferably 15x8x1,5 cm and more preferred 12x6x1,2 cm.

12. Kit according to at least one of the preceding claims, wherein the video device (3) is configured to provide visual or audio-visual instructions for using the medicament delivery device (4, 4a).

13. Kit according to at least one of the preceding claims, wherein the box (1) further comprises a medicament delivery device dummy (6).

14. Kit according to at least one of the preceding claims, wherein the box (1) further comprises a removable casing (5) for the at least one medicament delivery device (4, 4a) and/or the video device (3).

15. Kit according to claim 5, wherein the casing (5) comprises a first compartment (9) for receiving the at least one medicament delivery device (4, 4a) and a second compartment (10) for receiving the video device (3).
